# EUROPEAN PATENT APPLICATION

(11) **EP 4 595 887 A1**
(43) Date of publication of application: **06.08.2025**
(21) Application number: 23870759.0
(22) Date of filing: 25.09.2023
(51) Int. Cl.: A61B 6/03

(54) **STATIC CT IMAGING DEVICE WITH TUBE CURRENT MODULATION FUNCTION AND IMAGING METHOD THEREFOR**

(30) Priority: 26.09.2022 CN 202211174310
(71) Applicant: Nanovision Technology (Beijing) Co., Ltd., Beijing 100094 (CN)
(72) Inventor: CUI, Zhili, Beijing 100094 (CN); LI, Yunxiang, Beijing 100094 (CN); GAO, Jian, Beijing 100094 (CN)
(74) Representative: Wang, Bo
(86) International application number: PCT/CN2023/121283
(87) International publication number: WO 2024/067530

(57) **Abstract**

A static CT imaging device with a tube current modulation function and an imaging method therefor. The static CT imaging device comprises a ray source ring and a detector ring. A plurality of ray sources, which are uniformly distributed, are arranged on the ray source ring, and each ray source is configured to emit rays to scan a human body to be detected, and project positioning images of the human body at different angle positions onto the detector ring. A plurality of detectors, which are uniformly distributed, are arranged on the detector ring and configured to collect the rays to obtain the positioning images. According to the projection brightness of the positioning images, tube current modulation information about the ray source corresponding to the corresponding projection position is obtained, and the tube current modulation information comprises the numerical value of a tube current used by each ray source on the ray source ring during a medical examination. According to the tube current modulation information, the tube current of the corresponding ray source on the ray source ring is adjusted. Utilizing the static CT imaging device with the tube current modulation function and the imaging method therefor can achieve the automatic modulation of the tube current, thereby effectively reducing the irradiation dose to the human body.

## Description

### BACKGROUND

### Technical Field

The present invention relates to the technical field of medical instruments, and provides a static computed tomography (CT) imaging device with a tube current modulation function, and also provides a corresponding static CT imaging method.

### Related Art

CT is short for computed tomography. An imaging principle thereof is as follows: An X-ray beam and a highly sensitive X-ray detector are used to perform layer-by-layer axial scanning about a specific part of a human body. The X-ray passing through a layer is received by a scintillant on the X-ray detector, and is converted into visible light, which is then converted into an electric signal by a photoelectric converter, which is amplified, and then converted into a digital signal through an analog/digital converter, which is inputted into a computer for processing. A computer performs calculation on the information obtained through the layer-by-layer axial scanning to obtain an X-ray attenuation coefficient or an absorption coefficient of each voxel, which are then arranged into a matrix, namely, a digital voxel matrix. Digital information in the digital voxel matrix is converted into small blocks with a gray value ranging from black to white, which are called pixels in two-dimensional projection. The small squares are arranged in the form of a fault to form a CT image.

To improve a scanning speed, improve an imaging precision and speed, avoid impact of a centrifugal force from mechanical rotation, and reduce signal trailing effects and overlapping crosstalk during high-speed rotation, a static real-time CT imaging system is disclosed in the Chinese Patent No. CN105361900B. The static real-time CT imaging system includes a ring-shaped photon-counting detector, a ring-shaped X-ray scanning source, and a scanning sequence controller. The scanning sequence controller controls the ring-shaped X-ray scanning source to emit a narrow X-ray beam, which penetrates a to-be-examined object and is then projected onto a corresponding ring-shaped photon-counting detector. The ring-shaped photon-counting detector transmits corresponding exposure information to a data collection and processing unit and a human-machine interaction unit through a scanning host and a main control unit. Image reconstruction is completed in the data collection and processing unit and the human-machine interaction unit. During the scanning by the above static real-time CT imaging system, the ring-shaped X-ray scanning source does not need to rotate by a large degree. Instead, the X-ray is electronically controlled to switch to projection positions successively, which improves the scanning speed by tens of times, and can obtain a dynamic three-dimensional image. Using the photon-counting detector can obtain absorption data and energy data, thereby achieving real-time data reconstruction.

However, during a medical examination by using the CT device, a radiation dose needs to be controlled to reduce harm to a human body. Because most spiral CT devices has only one radiation source, in a most extreme case (chest scanning), one scanning rotation requires two tube current adjustments. If four scanning rotations are performed per second, a tube current adjustment frequency is about 8 Hz. For a hot cathode radiation source, a magnitude of a tube current is significantly affected by an ambient temperature and a filament temperature, resulting in very high technical difficulty in tube current modulation.

### SUMMARY

A primary technical problem to be resolved in the present invention is to provide a static computed tomography (CT) imaging device with a tube current modulation function.

Another technical problem to be resolved in the present invention is to provide a corresponding static CT imaging method.

To achieve the foregoing objectives, the present invention adopts the following technical solutions:
According to a first aspect of embodiments of the present invention, a static CT imaging device with a tube current modulation function is provided, including a radiation source ring and a detector ring.

The radiation source ring is provided with a plurality of radiation sources distributed uniformly. Each of the radiation sources is configured to emit a ray to scan a to-be-examined human body and project a scout image of the human body at a different angular position onto the detector ring. The detector ring is provided with a plurality of detectors distributed uniformly and configured to collect the ray to obtain the scout image.

Tube current modulation information of the radiation source corresponding to a corresponding projection position is obtained based on a projection brightness of the scout image. The tube current modulation information includes a value of a tube current to be used on each radiation source on the radiation source ring during a medical examination.

The tube current of the corresponding radiation source on the radiation source ring is adjusted based on the tube current modulation information.

Preferably, a 0-degree tube current to be used on a radiation source at a 0-degree position on the radiation source ring during the medical examination is obtained based on the projection brightness of the scout image.

A 90-degree tube current to be used on a radiation source at a 90-degree position on the radiation source ring during the medical examination is obtained based on a thickness of the human body determined based on a bed height and a coverage of the human body in the scout image in an X-Y direction.

Based on a symmetry principle, a 180-degree tube current is obtained based on the 0-degree tube current, and a 270-degree tube current is obtained based on the 90-degree tube current.

Interpolation is performed among the 0-degree tube current, the 90-degree tube current, the 180-degree tube current, and the 270-degree tube current by using an interpolation method, to obtain a tube current to be used on the radiation source at each position on the radiation source ring during the medical examination.

Preferably, the radiation sources alternately emit a ray at a set frequency, and projection areas corresponding to the radiation sources do not overlap.

Further, the static CT imaging device further includes an image processing apparatus. The image processing apparatus is connected to the detector ring, and the image processing apparatus is configured to receive the scout image and perform image processing on the scout image.

Preferably, the interpolation method is a linear interpolation method or an elliptical interpolation method.

According to a second aspect of the embodiments of the present invention, a static CT imaging method is provided, which is implemented based on the foregoing static CT imaging device, and includes the following steps:
emitting, by each radiation source on the radiation source ring, a ray to scan a to-be-examined human body, and projecting a scout image of the human body at a different angular position onto the detector ring, and obtaining, by a detector on the detector ring, the scout image;
obtaining tube current modulation information of the radiation source corresponding to a corresponding projection position based on a projection brightness of the scout image, where the tube current modulation information includes a value of a tube current to be used on each radiation source on the radiation source ring during a medical examination; and
adjusting the tube current of the corresponding radiation source on the radiation source ring based on the tube current modulation information.

Preferably, a 0-degree tube current to be used on a radiation source at a 0-degree position on the radiation source ring during the medical examination is obtained based on the projection brightness of the scout image.

A 90-degree tube current to be used on a radiation source at a 90-degree position on the radiation source ring during the medical examination is obtained based on a thickness of the human body determined based on a bed height and a coverage of the human body in the scout image in an X-Y direction.

Based on a symmetry principle, a 180-degree tube current is obtained based on the 0-degree tube current, and a 270-degree tube current is obtained based on the 90-degree tube current.

Interpolation is performed among the 0-degree tube current, the 90-degree tube current, the 180-degree tube current, and the 270-degree tube current by using an interpolation method, to obtain a tube current to be used on the radiation source at each position on the radiation source ring during the medical examination.

Preferably, the value of the tube current is associated with the thickness of the human body.

Compared with the prior art, the static CT imaging device with a tube current modulation function provided in the present invention configures, based on different thicknesses by which a ray passes through a human body at different projection angles, tube currents of the radiation sources at corresponding projection angles as values associated with the thicknesses of the human body, to achieve automatic tube current modulation, thereby effectively reducing a radiation dose to the human body.

### BRIEF DESCRIPTION OF THE DRAWINGS

FIG. 1 is an overall schematic structural diagram of a static computed tomography (CT) imaging device with a tube current modulation function according to an embodiment of the present invention.
FIG. 2 is a schematic diagram of a distribution of radiation sources in the static CT imaging device according to an embodiment of the present invention.
FIG. 3 is a schematic diagram of tube currents corresponding to projection angles according to an embodiment of the present invention.
FIG. 4 is a flowchart of a static CT imaging method with a tube current modulation function according to an embodiment of the present invention.

### DETAILED DESCRIPTION

Technical content of the present invention is described in detail below with reference to drawings and specific embodiments.

During a medical examination using a computed tomography (CT) device, automatic tube current modulation, to be specific, automatically adjusting a value of a tube current used during scanning based on a scout image, is a key technology for achieving radiation dose control. For an existing spiral CT device having only one radiation source, very high technical difficulty exists in tube current modulation.

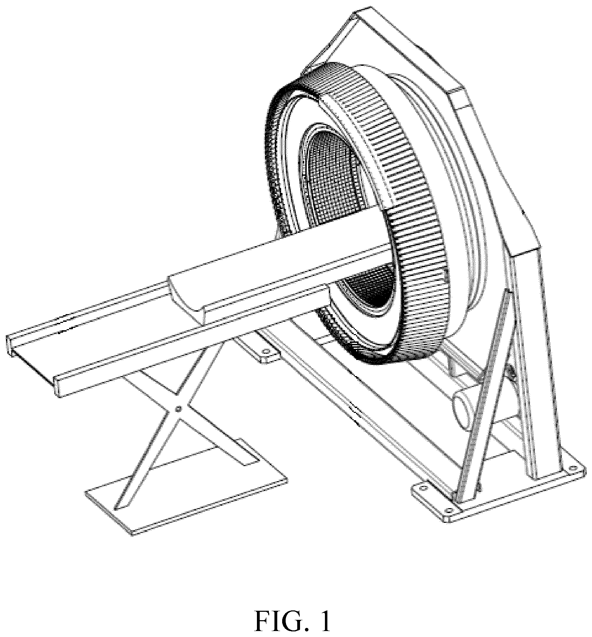

As shown in FIG. 1, a static CT imaging device is equipped with a plurality of radiation sources uniformly distributed in a circle, which, therefore, achieves an automatic tube current modulation technology completely different from the spiral CT device. In the static CT imaging device, real-time adjustment of a tube current of a single radiation source is not needed. Instead, only a tube current of a radiation source at a specific projection angle needs to be calculated based on a relationship between a tube current and a projection angle and a corresponding configuration needs to be performed. Before scanning, a tube current modulation solution pre-obtained through calculation for all angular positions are configured onto radiation sources at the corresponding angular positions. In this way, the static CT imaging device can perform scanning based on the configured tube current modulation solution.

Therefore, a unique automatic tube current modulation function can be achieved in combination with unique characteristics of a radiation source ring and a detector ring of the static CT imaging device. To be specific, based on different thicknesses by which an X-ray penetrates a human body at different projection angles, tube currents of radiation sources at corresponding projection angles are configured as values associated with the thicknesses of the human body, to achieve "automatic tube current modulation", which can effectively reduce a radiation dose to the human body.

It should be noted that an intensity of an X-ray is usually represented by a milliampere (mA) value of a tube current in the art. A brightness of a projection image (such as a scout image) may be controlled through control of value of a tube current. In addition, a 0-degree position in the embodiments of the present invention refers to a position directly above the radiation source ring and/or the detector ring angular position. Corresponding angular positions are calculated in a counterclockwise direction.

An embodiment of the present invention first provides a static CT imaging device with a tube current modulation function. A core idea of the static CT imaging device is to calculate, based on a relationship between a tube current and a projection angle, a value of a tube current required for a radiation source at a specific projection angle and to perform a configuration by using unique characteristics of a radiation source ring and a detector ring of the static CT imaging device. Before scanning, a tube current modulation solution pre-obtained through calculation for all angular positions are configured onto radiation sources at corresponding angular positions. Then scanning is performed based on the configured tube current modulation solution.

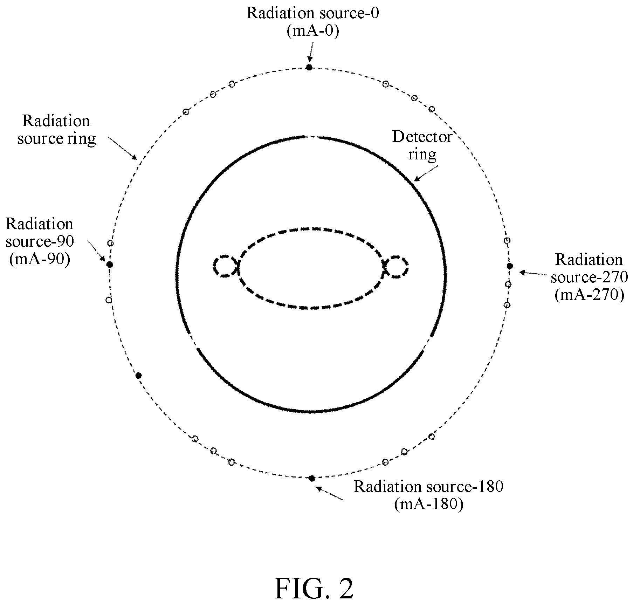

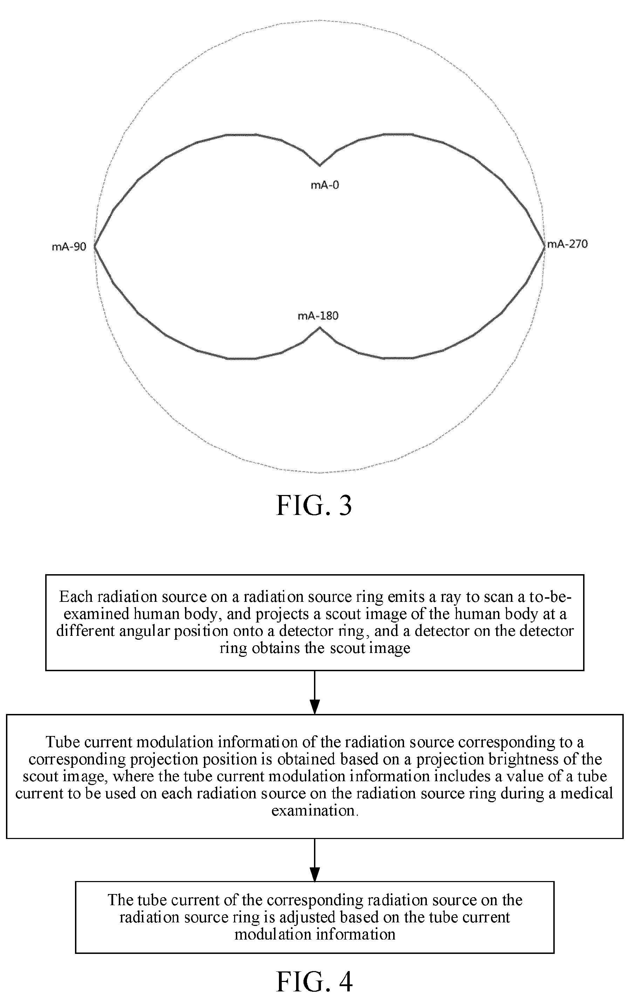

A specific implementation of the static CT imaging device with a tube current modulation function is described in detail with reference to the embodiments shown in FIG. 2 and FIG. 3.

As shown in FIG. 2, the static CT imaging device with a tube current modulation function includes a radiation source ring and a detector ring. The radiation source ring is provided with a plurality of radiation sources distributed uniformly. Each of the radiation sources is configured to emit a ray to scan a to-be-examined human body and project a scout image of the human body at a different angular position onto the detector ring. The detector ring is provided with a plurality of detectors distributed uniformly and configured to collect the ray to obtain the scout image. Tube current modulation information of the radiation source corresponding to a corresponding projection position is obtained based on a projection brightness of the scout image. The tube current modulation information includes a value of a tube current to be used on each radiation source on the radiation source ring during a medical examination. The tube current of the corresponding radiation source on the radiation source ring is adjusted based on the tube current modulation information.

In an embodiment of the present invention, the radiation source is preferably an X-ray source, or may be a radiation source of another type. A radiation source at a 0-degree position is used to scan to obtain a scout image of the human body, and a 0-degree tube current mA-0 to be used on the radiation source at the 0-degree position on the radiation source ring during the medical examination is obtained based on the projection brightness of the scout image.

A 90-degree tube current mA-90 to be used on a radiation source at a 90-degree position on the radiation source ring during the medical examination is obtained based on a thickness of the human body determined based on a bed height and a coverage of the human body in the scout image in an X-Y direction.

Based on a human body symmetry principle, a 180-degree tube current mA-180 is obtained based on the 0-degree tube current, where a value of mA-180 is equal to a value of mA-0; and a 270-degree tube current mA-270 is obtained based on the 90-degree tube current, where a value of mA-270 is equal to a value of mA-90. Specifically, because a human body is inherently symmetric, if mA-0, mA-90, mA-180, and mA-270 are used as four endpoints, tube currents at different projection angles are axially and centrally symmetry.

Therefore, for the tube currents corresponding to the projection angles shown in FIG. 3, interpolation may be performed among the 0-degree tube current, the 90-degree tube current, the 180-degree tube current, and the 270-degree tube current by using an interpolation method, to obtain a tube current to be used on the radiation source at each angular position on the radiation source ring during the medical examination.

In addition to the foregoing linear interpolation method, other interpolation methods may also be used. For example, the human body is simulated as an ellipse, with a 0-degree position and a 90-degree position respectively corresponding to a minor axis and a major axis of the ellipse. In this way, paths at which a ray passes through the ellipse at different projection angles may be calculated, thereby obtaining a tube current mA-X (X represents an angle) of a radiation source corresponding to each projection angle.

After configurations of the tube currents for the radiation sources at different projection angles are completed, a normal scanning process is initiated. The radiation sources at different projection angles on the radiation source ring perform medical examinations with different tube currents.

In an embodiment of the present invention, a plurality of (for example, 4, 6, or 8) radiation sources may be uniformly provided on the radiation source ring. The radiation sources alternately emit a ray at a set frequency which needs to ensure that projection areas corresponding to the radiation sources do not overlap to avoid interference.

In an embodiment of the present invention, the static CT imaging device may further include an image processing apparatus. The image processing apparatus is connected to the detector ring, and is configured to receive the scout image and perform image processing on the scout image.

Based on the foregoing static CT imaging device, as shown in FIG. 4, an embodiment of the present invention further provides a static CT imaging method with a tube current modulation function, specifically including steps S1 to S3.

S1: Each radiation source on the radiation source ring emits a ray to scan a to-be-examined human body, and projects a scout image of the human body at a different angular position onto the detector ring, and a detector on the detector ring obtains the scout image.

S2: Tube current modulation information of the radiation source corresponding to a projection position is obtained based on a projection brightness of the scout image of the human body, where the tube current modulation information includes a value of a tube current to be used on each radiation source on the radiation source ring during a medical examination.

S3: The tube current of the corresponding radiation source on the radiation source ring is adjusted based on the tube current modulation information.

Specifically, a radiation source at a 0-degree position is used to scan to obtain a scout image of the human body, and a 0-degree tube current mA-0 to be used on the radiation source at the 0-degree position on the radiation source ring during the medical examination is obtained based on the projection brightness of the scout image.

A 90-degree tube current mA-90 to be used on a radiation source at a 90-degree position on the radiation source ring during the medical examination is obtained based on a thickness of the human body determined based on a bed height and a coverage of the human body in the scout image in an X-Y direction.

Based on a human body symmetry principle, a 180-degree tube current mA-180 is obtained based on the 0-degree tube current, where a value of mA-180 is equal to a value of mA-0; and a 270-degree tube current mA-270 is obtained based on the 90-degree tube current, where a value of mA-270 is equal to a value of mA-90. To be specific, because a human body is inherently symmetric, if mA-0, mA-90, mA-180, and mA-270 are used as four endpoints, tube currents at different projection angles are axially and centrally symmetry.

Therefore, interpolation may be performed among the 0-degree tube current, the 90-degree tube current, the 180-degree tube current, and the 270-degree tube current by using an interpolation method, to obtain a value of a tube current to be used on the radiation source at each angular position on the radiation source ring during the medical examination. In different embodiments of the present invention, the interpolation method may be a linear interpolation method or an elliptical interpolation method.

Since the value of the tube current is associated with the thickness of the human body, the value of the tube current associated with the thickness of the human body is configured onto the corresponding radiation source on the radiation source ring. After configurations of the tube currents for the radiation sources at different projection angles are completed, a normal scanning process is initiated. The radiation sources at different projection angles on the radiation source ring perform medical examinations with different tube currents.

Compared with the prior art, in the static CT imaging device with a tube current modulation function and the imaging method therefor provided in the present invention, based on different thicknesses by which a ray passes through the human body at different projection angles, tube currents of the radiation sources at corresponding projection angles can be configured as values associated with the thicknesses of the human body, to achieve automatic tube current modulation, thereby effectively reducing a radiation dose to the human body.

The static CT imaging device with a tube current modulation function and the imaging method therefor provided in the present invention have been described in detail above. A person of ordinary skill in the art making any apparent change made to the present invention without departing from the essential content of the present invention constitutes infringement to the patent right of the present invention, and should bear a corresponding legal liability.

## Claims

1. A static computed tomography (CT) imaging device with a tube current modulation function, comprising a radiation source ring and a detector ring, wherein
the radiation source ring is provided with a plurality of radiation sources distributed uniformly, and each of the radiation sources is configured to emit a ray to scan a to-be-examined human body and project a scout image of the human body at a different angular position onto the detector ring; the detector ring is provided with a plurality of detectors distributed uniformly and configured to collect the ray to obtain the scout image;
tube current modulation information of the radiation source corresponding to a corresponding projection position is obtained based on a projection brightness of the scout image, wherein the tube current modulation information comprises a value of a tube current to be used on each radiation source on the radiation source ring during a medical examination; and
the tube current of the corresponding radiation source on the radiation source ring is adjusted based on the tube current modulation information.

2. The static CT imaging device with a tube current modulation function according to claim 1, wherein
a 0-degree tube current to be used on a radiation source at a 0-degree position on the radiation source ring during the medical examination is obtained based on the projection brightness of the scout image;
a 90-degree tube current to be used on a radiation source at a 90-degree position on the radiation source ring during the medical examination is obtained based on a thickness of the human body determined based on a bed height and a coverage of the human body in the scout image in an X-Y direction; and
based on a symmetry principle, a 180-degree tube current is obtained based on the 0-degree tube current, and a 270-degree tube current is obtained based on the 90-degree tube current.

3. The static CT imaging device with a tube current modulation function according to claim 2, wherein
interpolation is performed among the 0-degree tube current, the 90-degree tube current, the 180-degree tube current, and the 270-degree tube current by using an interpolation method, to obtain a tube current to be used on the radiation source at each position on the radiation source ring during the medical examination.

4. The static CT imaging device with a tube current modulation function according to claim 3, wherein
the interpolation method is a linear interpolation method or an elliptical interpolation method.

5. The static CT imaging device with a tube current modulation function according to claim 1, wherein
the radiation sources alternately emit a ray at a set frequency, and projection areas corresponding to the radiation sources do not overlap.

6. The static CT imaging device with a tube current modulation function according to claim 1, further comprising an image processing apparatus, wherein the image processing apparatus is connected to the detector ring, and the image processing apparatus is configured to receive the scout image and perform image processing on the scout image.

7. A static computed tomography (CT) imaging method, implemented based on the static CT imaging device according to any one of claims 1 to 6, and comprising the following steps:
emitting, by each radiation source on the radiation source ring, a ray to scan a to-be-examined human body, and projecting a scout image of the human body at a different angular position onto the detector ring, and obtaining, by a detector on the detector ring, the scout image;
obtaining tube current modulation information of the radiation source corresponding to a corresponding projection position based on a projection brightness of the scout image, wherein the tube current modulation information comprises a value of a tube current to be used on each radiation source on the radiation source ring during a medical examination; and
adjusting the tube current of the corresponding radiation source on the radiation source ring based on the tube current modulation information.

8. The static CT imaging method according to claim 7, wherein
a 0-degree tube current to be used on a radiation source at a 0-degree position on the radiation source ring during the medical examination is obtained based on the projection brightness of the scout image;
a 90-degree tube current to be used on a radiation source at a 90-degree position on the radiation source ring during the medical examination is obtained based on a thickness of the human body determined based on a bed height and a coverage of the human body in the scout image in an X-Y direction; and
based on a symmetry principle, a 180-degree tube current is obtained based on the 0-degree tube current, and a 270-degree tube current is obtained based on the 90-degree tube current.
interpolation is performed among the 0-degree tube current, the 90-degree tube current, the 180-degree tube current, and the 270-degree tube current by using an interpolation method, to obtain a tube current to be used on the radiation source at each angular position on the radiation source ring during the medical examination.

9. The static CT imaging method according to claim 8, wherein
the interpolation method is a linear interpolation method or an elliptical interpolation method.

10. The static CT imaging method according to any one of claims 7 to 9, wherein
the value of the tube current is associated with the thickness of the human body.
